## Europäisches Patentamt

## European Patent Office

## Office européen des brevets

(11) Veröffentlichungsnummer: **0 382 033 A2**

# EUROPÄISCHE PATENTANMELDUNG

(21) Anmeldenummer: **90101680.8**

(22) Anmeldetag: **28.01.90**

(51) Int. Cl.5: **A61K 6/083**

(30) Priorität: **09.02.89 DE 3903734**

(43) Veröffentlichungstag der Anmeldung:
**16.08.90 Patentblatt 90/33**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Heid, Renate, Dr.**
**Buchenweg 2**
**D-6803 Edingen(DE)**
Erfinder: **Winkel, Jens, Dr.**
**Letterhausstrasse 1**
**D-5000 Köln(DE)**
Erfinder: **Herold, Heiko, Dipl.-Ing.**
**Feuerdornweg 3**
**D-4040 Neuss 21(DE)**
Erfinder: **Schwabe, Peter, Dr.**
**Dudweiler Strasse 17**
**D-5090 Leverkusen(DE)**
Erfinder: **Finger, Werner, Prof.Dr.**
**Zonser Strasse 78**
**D-4047 Dormagen(DE)**
Erfinder: **Podszun, Wolfgang, Dr.**
**Roggendorfstrasse 55**
**D-5000 Köln 80(DE)**

(54) **Hybrid-Kunststoffüllungsmaterialien.**

(57) Die Erfindung betrifft neue lichthärtende Dentalmassen, insbesondere Zahnfüllungsmassen (composites) mit langer Lagerfähigkeit und Verarbeitungszeit.

EP 0 382 033 A2

## Hybrid-Kunststoffüllungsmaterialien

Die Erfindung betrifft neue lichthärtende Dentalmassen, insbesondere Zahnfüllungsmassen (composites).

Lichthärtende Zahnfüllungsmaterialien sind bekannt. Sie bestehen im allgemeinen aus ein oder mehreren (Meth)Acrylsäureharzen, oberflächenbehandelten Füllstoffen, Photoaktivatoren, Beschleunigern und Hilfsstoffen (z.B. Farbstoffe, Lichtschutzmittel, Stabilisatoren u.ä.). Man unterteilt die Füllungsmaterialien in solche, die im Frontzahn verwendet werden und solche, die im Seitenzahn eingesetzt werden. Letztere enthalten häufig große Mengen anorganischen Füllstoffs, um eine ausreichende Verschleißfestigkeit sowie gute mechanische Eigenschaften zu erreichen.

Wesentliche Voraussetzung für ein geeignetes Material ist eine genügend lange Verarbeitungszeit unter Umgebungslicht (Tageslicht).

Dem Zahnarzt muß genügend Zeit zur Verfügung stehen, um das Material in die Kavität einzubringen und dort seinen Vorstellungen entsprechend zu modellieren. Auch bei einer längeren Behandlungsdauer durch den Zahnarzt, z. B. beim Kantenaufbau, darf das Kunststoffüllungsmaterial nicht unter Umgebunglicht fest werden.

Auf der anderen Seite wird eine schnelle und vollständige Aushärtung der Füllung beim Belichten mit einer Dentallampe erwarteit. Die Polymerisationstiefen müssen also so groß wie möglich sein und gleichzeitig darf sich keine Inhibitionsschicht auf der Oberfläche des Materials bilden.

Zur Zeit sind keine Composites bekannt, die beiden Anforderungen genügen.

Viele der bekannten Füllungsmaterialien zeigen eine viel zu kurze Verarbeitungszeit. Überprüft man diese Materialien in einem Test, wie er z.B. im DIN-Vorschlag 0013922 vorgesehen ist, so sind sie häufig nur bis zu 10 Sekunden verarbeitbar. Das ist vor allem für Füllungen im molaren Bereich viel zu kurz, da die Gefahr besteht, daß das Material während der Verarbeitung aushärtet bzw. anpolymerisiert und so -für den Zahnarzt zum Teil nicht erkennbar - geschädigt wird. Andere Materialien weisen zwar eine ausreichende Verarbeitungszeit auf, bilden jedoch eine tiefe Inhibitionsschicht an der Oberfläche. Überprüfung der Materialien in einem Test, wie er in der "Revision of ISO 4049, 1978" vorgesehen ist, ergeben bei diesem Materialien starke Oberflächenverfärbungen. Hinzu kommt, daß die Füllungsmaterialien häufig schon nach sehr kurzer Lagerzeit im Aushärtungsverhalten nachlassen und dem Zahnarzt keine Garantie für vollständige Aushärtung bieten.

Die der vorliegenden Erfindung zugrundeliegende Aufgabe war es, ein lagerfähiges lichthärtbares Kunststoffüllungsmaterial zur Verfügung zu stellen, bei dessen Verwendung einerseits genügend Zeit bleibt, um das Material sorgfältig zu plazieren, es aber andererseits beim Belichten mit einer handelsüblichen Dentallampe schnell und vollständig aushärtet.

Es wurde gefunden, daß die Verwendung von p-Dialkylaminobenzolsulfonsäureamiden als Beschleuniger zusammen mit Campherchinon in Kombination mit oberflächenbelegten Glasfüllstoffen eines bestimmten Silanisierungsgrades und Mikrofillern sowie handelsüblichen Monomeren und Hilfsstoffen Füllungsmaterialien ergaben, die lange verarbeitbar sind, andererseits aber nach dem Belichten mit einer Dentallampe schnell und vollständig aushärten. Im Vergleich zu bekannten Produkten zeigen diese Materialien nach 2-jähriger Lagerung keine Verschlechterung der mechanischen Eigenschaften.

Die erfindungsgemäßen lichthärtenden Dentalmassen, insbesondere Zahnfüllungsmassen, enthaltend eine oder mehrere polymerisierbare (Meth)acrylverbindungen, Photoinitiatoren, Beschleuniger, anorganische Füllstoffe und gegebenenfalls übliche Hilfsstoffe, sind dadurch gekennzeichnet, daß sie

a) 10 - 50 Gew.% eines oder mehrerer, mindestens 2 polymerisierbare (Meth)Acrylsäuregruppen enthaltende Monomere,

b) 5 - 80 Gew.% eines Glases oder einer Glaskeramik mit einem mittleren Teilchendurchmesser zwischen 0,1 und 10 µm, oberflächenbehandelt mit γ-Methacryloxypropyltrimethoxysilan bis zu einem Kohlenstoffgehalt zwischen 0,2 bis 2,5 Gew.%,

c) 2 - 10 Gew.% eines oberflächenbehandelten Mikofüllers und

d) 0,1 - 5 Gew.% eines Photoaktivators, bestehend aus einer Mischung von Campherchinon und p-Dialkylaminobenzolsulfonsäureamid enthalten.

Beispielsweise seien die folgenden Monomere genannt:

Triethylenglycoldimethacrylat,Tetraetylenglycoldimethacrylat, 1,12-Dodecandioldimethacrylat, 1,6-Hexandioldimethacrylat, Dietylenglycoldimethacrylat, 2,2-Bis(p-(2′-hydroxy-3′-methacryloyloxypropoxy)phenyl)-propan, 2,2-Bis(p-(2′-methacryloyloxyethoxy)phenyl)propan, Trimethylolpropan-tri(meth)acrylat, Bis-(Meth)-acryloyloxymethyl)-tricyclo[5.2.1.0$^{2.6}$]decan (gemäß DE-OS 2 931 925 und DE-OS 2 931 926), 1,3-Di((meth)-

2

acryloyloxypropyl)-1,1,3,3-tetra-methyldisiloxan,     1,3-Bis(3-(meth)acryloyloxyethylcarbamoyloxy-propyl)-1,1,3,3-tetra-methyldisiloxan.

Insbesondere werden Monomere bevorzugt, die bei 13 mbar einen Siedepunkt von über 100 ° C besitzen.

Im Rahmen der vorliegenden Erfindung ist es bevorzugt, Mischungen verschiedener erfindungsgemäßer (Meth)acrylsäureester einzusetzen.

Es ist auch möglich, Monomermischungen einzusetzen, die mehrere Comonomere enthalten.

Bevorzugte erfindungsgemäße lichthärtende Dentalmassen, insbesondere Zahnfüllungsmassen, enthaltend ein oder mehrere polymerisierbare (Meth)Acrylverbindungen, Photoinitiatoren, Beschleuniger, anorganische Füllstoffe und gegebenenfalls übliche Hilfsstoffe, sind dadurch gekennzeichnet, daß sie

a) 10 - 30 Gew.% eines oder mehrerer, mindestens 2 polymerisierbare (Meth)Acrylsäuregruppen enthaltende Monomere,

b) 40 - 75 Gew.% eines Glases oder einer Glaskeramik mit einer Teilchengröße zwischen 0,1 und 10 μm, nachbehandelt mit γ-Methacryloxypropyltrimethoxysilan bis zu einem Kohlenstoffgehalt zwischen 0,5 bis 2,5 Gew.%,

c) 2 - 10 Gew.% eines oberflächenbehandelten Mikrofüllers und

d) 0,1 - 5 Gew.% eines Photoaktivators, bestehend aus einer Mischung von Campherchinon und p-Dialkylaminobenzolsulfonsäureamid enthalten.

Die nachfolgenden Beispiele demonstrieren die Vorzüge der erfindungsgemäßen Composites.

Wie bereits erwähnt, ist es für ein Kunststoffüllungsmaterial wesentlich, daß es eine genügend lange Verarbeitungszeit bei gleichzeitig schneller und vollständiger Aushärtung beim Belichten mit einer Dentallampe aufweist. Die Polymerisationstiefen sollten also möglichst groß sein. Von Nachteil ist eine Inhibitionsschicht an der Oberfläche des ausgehärteten Kunststoffes, die sich als starke Oberflächenfärbung bei einem Test nach "Revision of ISO 4049/1978" bemerkbar macht; sie muß vermieden werden. Das optimale Composite sollte also lange Verarbeitungszeiten bei Umgebungslicht, eine schnelle und vollständige Durchhärtung beim Belichten mit einer Dentallampe und keine Oberflächenfärbung aufweisen. Diesem Anspruch genügen die erfindungsgemäßen Kunststoffüllungsmaterialien.

Beispiel 1 (Vergleichsbeispiel)

| In 1000 g Paste sind enthalten: | |
|---|---|
| Bisphenol-A-diglycidyldimethacrylat (BIS GMA): | 128,725 g |
| Trietylenglycoldimethacrylat (TEGDMA): | 105,330 g |
| oberflächenbehandelter Ba-Glasfüllstoff, mittlerer Teilchendurchmesser 1,3 μm | 762,225 g |
| Campherchinon/Diethylaminoethylacrylat als Startersystem: | 1,010 g |
| Hilfs- und Zusatzstoffe: (2,38 g ®TINUVIN P, 0,33 g ®IONOL) | 2,710 g |
| Verarbeitungszeit der Paste bei Umgebungslicht gemessen nach DIN-Vorschlag 0013922: | 150 s |
| Polymerisationstiefe nach 60 s Belichtungszeit mit einer Dentallampe: | 6,5 mm |
| Inhibitionsschicht an der Oberfläche des ausgehärteten Kunststoffüllungsmaterials bestimmt nach einem Test, wie er in der "Revision of ISO 4049/1978" vorgesehen ist: | ja |

Beispiel 2 (Vergleichsbeispiel)

| In 1000 g Paste sind enthalten: | |
| --- | --- |
| BIS GMA: | 62,00 g |
| TEGDMA: | 62,00 g |
| oberflächenbehandelter Zn-Glasfüllstoff, mittlerer Teilchendurchmesser 2,3 µm | 875,00 g |
| Hilfs- und Zusatzstoffe (1,26 g ®TINUVIN P, 0,18 g ®IONOL) | 1,44 g |
| Campherchinon N,N-Dimethylamino-p-hydroxyethylbenzol als Startersystem: | 1,30 g |
| Verarbeitungszeit der Paste bei Umgebungslicht, gemessen nach DIN-Vorschlag 0013922: | 50 s |
| Polymerisationstiefe nach 60 s Belichtungszeit mit einer Dentallampe: | 5,9 mm |
| Inhibitionsschicht an der Oberfläche des ausgehärteten Kunststofffüllungsmaterials bestimmt nach einem Test, wie er in er "Revision of ISO 4049/1978" vorgesehen ist: | ja |

Beispiel 3 (Vergleichsbeispiel)

| In 1000 g Paste sind enthalten: | |
| --- | --- |
| BIS Phenol-A PO/Hexamethylendiisocyanat: | 74,25 g |
| TEGDMA: | 60,75 g |
| oberflächenbehandelter Ba-Glasfüllstoff, mittlerer Teilchendurchmesser 3 µm: | 862,03 g |
| Hilfs- und Zusatzstoffe: (1,37 g ®TINUVIN P, 0,19 g ®IONOL) | 1,56 g |
| Campherchinon/Dimethylaminoethylmethacrylat als Startersystem: | 1,41 g |
| Verarbeitungszeit der Paste bei Umgebungslicht gemessen nach DIN-Vorschlag 0013922: | 30 s |
| Polymerisationstiefe nach 60 s Belichtungszeit mit einer Dentallampe: | 6,5 mm |
| Inhibitionsschicht an der Oberfläche des ausgehärteten Kunststofffüllungsmaterials bestimmt nach einem Test, wie er in der "Revision of ISO 4049/1978" vorgesehen ist: | nein |

Beispiel 4 Vergleichsbeispiel)

| In 1000 g Paste sind enthalten: | |
| --- | --- |
| BIS GMA: | 71,00 g |
| TEGDMA: | 71,00 g |
| oberflächenbehandelter Zr-Keramikfüllstoff, mittlerer Teilchendurchmesser 2 µm: | 854,83 g |
| Hilfs- und Zusatzstoffe: (1,45 g ®TINUVIN P, 0,20 g ®IONOL) | 1,65 g |
| Campherchinon/N,N-Dimethylamino-p-hydroxyethylbenzol (enthalten im Monomer) | 1,52 g |
| Verarbeitungszeit der Paste bei Umgebungslicht gemessen nach DIN-Vorschlag 0013922: | 15 s |
| Polymerisationstiefe bei 60 s Belichtungszeit mit einer Dentallampe: | 4,7 mm |
| Inhibitionsschicht an der Oberfläche des ausgehärteten Kunststofffüllungsmaterials bestimmt nach einem Test, wie er in der "Revision of ISO 4049/1978" vorgesehen ist: | nein |

Beispiel 5 (erfindungsgemäß)

4

| In 1000 g Paste sind enthalten: | |
|---|---|
| BIS GMA: | 172,015 g |
| TEGDMA: | 74,008 g |
| oberflächenbehandelter Ba-Glasfüllstoff, mittlerer Teilchendurchmesser 1,2 μm: | 749,486 g |
| Campherchinon/Diallylsulfonamid als Startersystem: | 1,641 g |
| Hilfs- und Zusatzstoffe: (2,50 g ®TINUVIN P, 0,35 g ®IONOL) | 2,850 g |
| Verarbeitungszeit der Paste bei Umgebungslicht gemessen nach DIN-Vorschlag 0013922: | 100 s |
| Polymerisationstiefe bei 60 s Belichtungszeit mit einer Dentallampe: | 7,7 mm |
| Inhibitionsschicht an der Oberfläche des ausgehärteten Kunststoffüllungsmaterials bestimmt nach einem Test, wie er in der "Revision of ISO 4049/1978" vorgesehen ist: | nein |

Die aufgezeigten Beispiele lassen deutlich erkennen, daß das erfindungsgemäße Composite (Beispiel 5) mit dem neuen Startersystem Campherchinon/Diallylsulfonamid in Kombination mit den oberflächenbelegten Glasfüllstoffen eines bestimmten Silanisierungsgrades die gestellten Anforderungen erfüllt. Neben einer langen Verarbeitungszeit bei Umgebungslicht wird eine schnelle und vollständige Durchhärtung bei Belichtung mit einer Dentallampe erreicht, während das ausgehärtete Kunststoffüllungsmaterial keine Oberflächenverfärbung zeigt.

Das Material gemäß Vergleichsbeispiel 1 weist zwar eine sehr lange Verarbeitungszeit bei Umgebungslicht auf, die Durchhärtungstiefen beim Belichten mit einer Dentallampe sind jedoch gering, und gleichzeitig bildet sich eine starke Inhibitionsschicht auf der Oberfläche des ausgehärteten Materials aus.

Die Materialien nach Beispiel 2,3 und 4 weisen sehr kurze Verarbeitungszeiten unter Umgebungslicht auf.

## Ansprüche

1. Dentalmassen, enthaltend eine oder mehrere polymerisierbare (Meth)-Acrylverbindung, Photoinitiatoren, Beschleuniger, anorganische Füllstoffe und übliche Hilfsstoffe, dadurch gekennzeichnet, daß sie

a) 10 - 50 Gew.% eines oder mehrerer, mindestens 2 polymerisierbare (Meth)-Acrylsäuregruppen enthaltende Monomere,

b) 5 - 80 Gew.% eines Glases oder einer Glaskeramik mit einer mittleren Teilchengröße zwischen 0,1 und 10 μm, nachbehandelt mit γ-Methacryloxypropyltrimethoxysilan bis zu einem Kohlenstoffgehalt zwischen 0,5 bis 2,5 Gew.%,

c) 2 - 10 Gew.% eines oberflächenbehandelten Mikrofüllers und

d) 0,1 - 5 Gew.% eines Photoaktivators, bestehend aus einer Mischung von Campherchinon und p-Dialkylaminobenzolsulfonsäureamid
enthalten.

2. Dentalmassen gemäß Anspruch 1, dadurch gekennzeichnet, daß sie

a) 10 - 30 Gew.% eines oder mehrerer, mindestens 2 polymerisierbare (Meth)-Acrylsäuregruppen enthaltende Monomere,

b) 40 - 75 Gew.% eines Glases oder einer Glaskeramik mit einer mittleren Teilchengröße zwischen 0,1 und 10 μm, nachbehandelt mit γ-Methacryloxypropyltrimethoxysilan bis zu einem Kohlenstoffgehalt zwischen 0,5 bis 2,5 Gew.%,

c) 2 - 10 Gew.% eines oberflächenbehandelten Mikrofüllers und

d) 0,1 - 5 Gew.% eines Photoaktivators, bestehend aus einer Mischung von Campherchinon und p-Dialkylaminobenzolsulfonsäureamid
enthalten.